(19) Europäisches Patentamt
European Patent Office
Office européen des brevets

(11) Veröffentlichungsnummer: **0 018 581**
**B1**

# (12) EUROPÄISCHE PATENTSCHRIFT

(45) Veröffentlichungstag der Patentschrift: **25.05.83**

(51) Int. Cl.³: **C 07 C 125/06**

(21) Anmeldenummer: **80102171.8**

(22) Anmeldetag: **23.04.80**

## (54) Verfahren zur Herstellung von Aryl-mono- und/oder -polyurethanen.

(30) Priorität: **30.04.79 DE 2917568**
**20.10.79 DE 2942511**

(43) Veröffentlichungstag der Anmeldung: **12.11.80 Patentblatt 80/23**

(45) Bekanntmachung des Hinweises auf die Patenterteilung: **25.05.83 Patentblatt 83/21**

(84) Benannte Vertragsstaaten: **AT BE CH DE FR GB IT LI NL SE**

(56) Entgegenhaltungen: **keine**

(73) Patentinhaber: **BASF Aktiengesellschaft, Carl-Bosch-Strasse 38, D-6700 Ludwigshafen (DE)**

(72) Erfinder: **Merger, Franz, Dr., Max-Slevogt-Strasse 25, D-6710 Frankenthal (DE)**
Erfinder: **Towae, Friedrich, Dr., Parkstrasse 22, D-6700 Ludwigshafen (DE)**
Erfinder: **Harder, Wolfgang, Dr., Loensstrasse 20, D-6940 Weinheim (DE)**

**EP 0 018 581 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Verfahren zur Herstellung von Aryl-mono- und/oder -polyurethanen

Die Erfindung betrifft ein Verfahren zur Herstellung von Aryl-mono- und/oder -polyurethanen durch Umsetzung von O-Alkylcarbamidsäureestern mit primären aromatischen Mono- und/oder Polyaminen in Gegenwart von Alkoholen und gegebenenfalls Harnstoff bei erhöhten Temperaturen und gegebenenfalls Abtrennung des entstehenden Ammoniaks. Zu Polyurethanen bzw. Polyaminen gehören im Sinne der Erfindung auch Diurethane bzw. Diamine.

N-Arylurethane werden technisch üblicherweise durch Umsetzung von Alkoholen mit aromatischen Isocyanaten oder von aromatischen Aminen mit Chlorkohlensäureestern hergestellt, wobei sowohl die Isocyanate als auch die Chlorkohlensäureester durch Phosgenierung der entsprechenden Amine und Abspaltung von Chlorwasserstoff oder durch Phosgenierung der Alkohole gewonnen werden. (Methoden der organischen Chemie, Houben-Weyl, Band 8, Seiten 137, 120 und 101, Georg Thieme Verlag, Stuttgart, 1952) Diese Verfahren sind technisch sehr aufwendig; ferner bringt die Verwendung von Phosgen wegen damit verbundener Sicherheits- und Umweltschutzmaßnahmen erhebliche Nachteile mit sich.

N-Arylurethane finden als Zwischen- und Endprodukte Verwendung. Gemäß DE-OS 2 635 490 oder US-PS 3 919 278 sind sie beispielsweise zur Herstellung von Isocyanaten geeignet. Andere Herstellungsverfahren für N-Substituierte Urethane gewinnen daher zunehmend an Bedeutung.

So beschreibt die DE-OS 2 160 111 ein Verfahren zur Herstellung von N-substituierten Urethanen durch Umsetzung eines organischen Carbonats mit einem primären oder sekundären aromatischen Amin in Gegenwart einer Lewissäure. Nachteilig an diesem Verfahren ist, daß organische Carbonate aus Phosgen oder Kohlenmonoxid und Alkohol hergestellt werden und daher teuer sind, die Reaktionsgeschwindigkeit recht gering und die Reaktionszeiten dementsprechend groß sind; außerdem werden als Nebenprodukte zusätzlich stets N-Alkyl-arylamine erhalten.

Nach Angaben der US-PS 2 834 799 werden Carbamidsäure- und Kohlensäureester durch Umsetzung von Harnstoffen mit Alkoholen in Gegenwart von Bortrifluorid hergestellt. Nachteilig ist hierbei, daß das Bortrifluorid als Katalysator in äquimolaren Mengen benötigt wird, so daß pro erzeugtes Molekül Carbamidsäureester mindestens ein Molekül Bortrifluorid verbraucht wird. Dadurch gestaltet sich das Verfahren nicht nur teuer, sondern es stellt auch eine erhebliche Umweltbelastung dar, da das Bortrifluorid in Form des $H_3N.BF_3$-Adduktes anfällt.

Methyl-N-phenylurethan kann ferner aus Kohlenmonoxid, Schwefel, Anilin und Methanol hergestellt werden (R. A. Franz et al, J. Org. Chem. 28, 585 (1963)). Nachteilig sind hierbei insbesondere die geringen Ausbeuten, die auch bei langen Reaktionszeiten nicht über 25% liegen.

Nach Angaben der US-PS 2 409 712 können N-Alkyl- und N-Arylmonourethane durch Umsetzung von Monoaminen mit Harnstoff, N,N'-Dialkyl- oder N,N'-Diarylharnstoff und Alkoholen bei Temperaturen von 150°C bis 350°C, gegebenenfalls unter erhöhtem Druck, hergestellt werden. Beispielhaft beschrieben wird jedoch lediglich die Herstellung von N-Alkylmonourethanen nach der genannten Methode, während N-Phenylurethan durch Alkoholyse von Diphenylharnstoff hergestellt wird.

Zur Herstellung von N-substituierten Monourethanen wird der Harnstoff gemäß US-PS 2 677 698 zunächst mit Monoaminen in den entsprechenden N,N'-disubstituierten Harnstoff übergeführt, dieser wird gereinigt und anschließend mit Alkohol zur Reaktion gebracht.

Nachteilig an den genannten Verfahren sind neben einer aufwendigen Technik insbesondere die geringen Ausbeuten, die auch durch die Herstellung und Reinigung von N,N'-disubstituierten Harnstoffen nicht verbessert werden können.

Diese Nachteile können auch mit Hilfe des Verfahrens nach US-PS 2 806 051 nicht beseitigt werden. Nach Angaben dieser Patentschrift wird beispielsweise Anilin mit Harnstoff und Alkohol im Molverhältnis 1,0 : 1,2 : 2,0 bei Temperaturen unter 200°C, vorzugsweise von 120° bis 160°C umgesetzt. Auch in den vorzugsweise genannten Temperaturbereichen werden in technisch zweckmäßigen Reaktionszeiten nach dieser Verfahrensweise N-Phenylmonourethane nur in geringen Ausbeuten erhalten. Es ist daher nicht überraschend, daß in der nachgängigen US-PS 3 076 007 zur Herstellung von N-Alkyl- und N-Cycloalkylurethanen die obengenannten Methoden unter Verwendung von gegebenenfalls substituierten Harnstoffen nicht beschrieben werden; erwähnt wird hingegen die Umsetzung von Phosgen mit Alkoholen zu Chloralkylformiaten und die anschließende Weiterreaktion mit Aminen zu Urethanen, während als besonders vorteilhaft zur Herstellung der Urethane die Reaktion von Aminen mit Äthylencarbonat empfohlen wird. Nach einer neueren Verfahrensvariante (DE-OS 2 716 540) werden zur Herstellung von aromatischen Urethanen Dialkylcarbonate mit N-Acylaminen zur Reaktion gebracht.

Es ist ferner bekannt, daß Carbamidsäureäthylester in siedendem Dioxan mit Aminen nicht reagieren (D. G. Crosby and C. Niemann, J. Am. Chem. Soc. 76 4458 (1954)), und daß N-Alkylurethane mit alkoholischer Ammoniaklösung bei Temperaturen von 160° bis 180°C eine alkalische Lösung ergeben, aus der nach Neutralisation mit Salzsäure Aminhydrochlorid, Harnstoff, Alkylharnstoff und Alkylurethan isoliert werden kann (M. Brander, Rec. trav. Chim 37 88–91 (1917).

Die genannten Publikationen enthalten keine Angaben über die Reaktion von aromatischen

primären Aminen mit Carbamidsäureestern, obwohl aus Ann. 147 (1868) 163 bekannt war, das durch gemeinsames Erhitzen von Carbamidsäureäthylester mit Anilin bei 160°C im Bombenrohr Diphenylharnstoff gebildet wird.

Nach Angaben der US-PS 24 09 712 erhält man aus aliphatischen Monoaminen, Harnstoff und Alkohol Alkylurethane nur in geringen Ausbeuten trotz des Einsatzes eines Harnstoffüberschusses. Da gemäß US-PS 2 806 051 bei niedrigerer Temperatur mit weniger Harnstoff etwas höhere Ausbeuten erzielt werden, mußte der Fachmann annehmen, daß höhere Molverhältnisse von Harnstoff zu Amin nachteilig sind. Als Nebenprodukte der Synthese von Phenylurethan wurden Diphenylharnstoff und O-Alkylcarbamidsäureester festgestellt. Der O-Alkylcarbamidsäureester wurde hierbei neben nicht umgesetztem Anilin durch Destillation isoliert. Die Bildung von O-Alkylcarbamidsäureester aus Harnstoff und Alkohol wurde demnach als störende Nebenreaktion angesehen.

Da bereits die Herstellung von N-monoalkylsubstituierten Urethanen aus Alkylaminen, Harnstoff und Alkoholen nur in mäßigen Ausbeuten gelingt und Carbamidsäureester als Nebenprodukte gebildet werden, ist nicht überraschend, daß durch keine der genannten Publikationen — weder einzeln noch in Kombination — eine Lehre vermittelt wird, die die Herstellung von Aryl-mono-, und/oder -polyurethanen aus Arylaminen und Carbamidsäureester in Betracht zieht. Zur Beseitigung dieser Nachteile wurde vorgeschlagen, N-Arylurethane aus Nitroaromaten, Kohlenmonoxid und Alkoholen in Gegenwart von Katalysatoren herzustellen:

Nach Angaben der DE-OS 1 568 044 (US-PS 3 467 694) können Urethane durch Umsetzung von organischen Nitroverbindungen, Kohlenmonoxid und hydroxylhaltigen Verbindungen in Gegenwart eines Katalysators, der aus einem Edelmetall und einer Lewissäure besteht, unter praktisch wasserfreien Bedingungen in Abwesenheit von Wasserstoff bei erhöhtem Druck und Temperaturen über 150°C hergestellt werden. Gemäß DE-OS 2 343 826 (US-PS 3 895 054) werden Urethane aus hydroxylgruppenhaltigen Verbindungen, Kohlenmonoxid und Nitro-, Nitroso-, Azo- und Azoxygruppen aufweisenden Verbindungen in Gegenwart von Schwefel, Selen, einer Schwefel- und/oder Selenverbindung und mindestens einer Base und/oder Wasser hergestellt. Die DE-OS 2 623 694 (US-PS 4 080 365) beschreibt die Herstellung aromatischer Urethane aus den oben genannten Ausgangsverbindungen in Gegenwart von Selen enthaltenden Katalysatorsystemen sowie speziellen aromatischen Amino- und Harnstoffverbindungen.

Aber auch diese Verfahren weisen noch schwerwiegende Nachteile auf. Das toxische Kohlenmonoxid und Katalysatoren, die toxisch sind bzw. im Laufe der Umsetzung toxische Verbindungen bilden, beispielsweise Selen- und Schwefelwasserstoff, oder Katalysatoren, die sehr teuer und schwierig rückführbar sind, wie Palladium, erfordern einen hohen technischen Aufwand und kostspielige Sicherheitsmaßnahmen.

Die Aufgabe der vorliegenden Erfindung bestand darin, Aryl-mono- und/oder -polyurethane aus leicht zugänglichen Ausgangskomponenten, möglichst in einer Reaktionsstufe unter wirtschaftlich vertretbaren Bedingungen in guten Ausbeuten herzustellen. Die Verwendung von stark toxischen Ausgangsstoffen, beispielsweise von Phosgen, Kohlenmonoxid, oder Katalysatoren, die toxisch sind oder im Laufe der Umsetzung toxische Verbindungen bilden, beispielsweise Schwefelwasserstoff, sollte weitgehend vermieden werden.

Diese Aufgabe wurde gelöst durch ein Verfahren zur Herstellung von Aryl-mono- und/oder -polyurethanen, das dadurch gekennzeichnet ist, daß man O-Alkylcarbamidsäureester mit primären aromatischen Mono- und/oder Polyaminen in Gegenwart von Alkoholen und gegebenenfalls Harnstoff bei erhöhten Temperaturen umsetzt und den entstehenden Ammoniak gegebenenfalls abtrennt.

Die Reaktion kann durch die Gleichung (I) veranschaulicht werden:

$$Ar(-NH_2)_n + n\,H_2N-COOR \xrightarrow[\Delta]{ROH} Ar(-NH-COOR)_n + n\,NH_3 \quad (I)$$

Vorzugsweise wird die Umsetzung jedoch in Gegenwart von Harnstoff nach Gleichung (II) durchgeführt:

$$Ar(-NH_2)_n + a\,H_2N-CO-NH_2 + b\,H_2N-COOR + a\,ROH \longrightarrow Ar(-NH-COOR)_n + (2a+b)NH_3 \quad (II)$$

In den Gleichungen (I) und (II) bedeuten n, a und b ganze Zahlen, wobei n für 1 bis 7, vorzugsweise 1 bis 5 steht und nach Gleichung (II) $a+b=n$ und $a:n=1{,}5-0$ sind.

Die Bildung von Aryl-mono- und/oder -polyurethanen, insbesondere in einem Verfahrensschritt und in guten Ausbeuten, war besonders überraschend, da nach bekannter Lehrmeigung aus Carbamidsäureestern und aromatischen Aminen N,N'-Diarylharnstoffe erhalten werden, die meist aus Alkohol umkristallisiert werden. Unsubstituierte Urethane reagieren in Gegenwart von aromatischen Aminen auch in Alkohol sehr leicht zu N,N'-Diarylharnstoffen weiter. (Methoden der organischen

Chemie, Houben-Weyl, Band 8, Seiten 152, 140 und 161, Georg Thieme Verlag, Stuttgart, 1952).

Entsprechend werden aus Diaminen und Harnstoff bei Temperaturen über 100°C hochmolekulare, verspinnbare Kondensationsprodukte erhalten (DE-PS 896 412). Hochmolekulare Polyharnstoffe, beispielsweise mit Molekulargewichten von 8000 bis 10 000 und größer werden auch erhalten, wenn man Diurethane mit Diaminen bei Temperaturen von ungefähr 150°C bis 300°C kondensiert (US-PS 2 181 663 und US-PS 2 568 885). Da Mono- und Polyurethane außerdem thermisch in Isocyanate, Alkohole und gegebenenfalls Olefine, Kohlendioxid, Harnstoff und Carbodiimide gespalten werden, wobei die erhaltenen Spaltprodukte wieder zahlreiche Folgeprodukte, z. B. Biurete, Allophanate, Isocyanurate, Polycarbodiimide u. a. bilden können, (J. A. C. S. 80, 5495 (1958) und J. A. C. S. 78, 1946 (1956)) konnte nicht erwartet werden, daß unter sehr ähnlichen Reaktionsbedingungen nach dem erfindungsgemäßen Verfahren Aryl-mono- und/oder -polyurethane in guten Ausbeuten erhalten werden.

Es ist insbesondere überraschend, daß O-Alkylcarbamidsäureester bzw. Mischungen aus O-Alkylcarbamidsäureester und Harnstoff in Gegenwart von Alkohol mit Primären aromatischen Aminen zu Arylurethanen reagieren, diese Reaktion in Abwesenheit eines Katalysators oberhalb des für Harnstoff bekannten optimalen Temperaturbereichs mit befriedigender Geschwindigkeit und hoher Selektivität durchführbar ist und überschüssige O-Alkylcarbamidsäureester und Harnstoff nicht zu wesentlichen Zersetzungen und (Poly-)kondensationsreaktionen führen.

Es ist nicht erforderlich O-Alkylcarbamidsäureester in einer vorgelagerten Verfahrensstufe getrennt herzustellen. In einer leicht praktikablen, vorzugsweise zur Anwendung kommenden Ausführungsform wird O-Alkylcarbamidsäureester zusammen mit Harnstoff und Alkohol eingesetzt und nach weitgehender bis vollständiger Umsetzung des aromatischen Mono- und/oder Polyamins durch Destillation abgetrennt und gegebenenfalls zurückgeführt. Das erfindungsgemäße Verfahren kann auch kontinuierlich durchgeführt werden.

Für die erfindungsgemäße Umsetzung mit O-Alkylcarbamidsäureestern in Gegenwart von Alkoholen und gegebenenfalls Harnstoff eignen sich gegebenenfalls substituierte primäre aromatische Mono-, Di- und Polyamine. Im einzelnen seien beispielhaft genannt:

Aromatische Monoamine, wie Anilin, substituierte Aniline, wie in 2-, 3- und/oder 4-Stellung durch eine Nitro-, Methyl-, n-Propyl-, Isopropyl-, n-Butyl-, Isobutyl-, sek.-Butyl-, tert.-Butylgruppe oder ein Chloratom substituierte Aniline; ortho-, meta- und/oder para-Hydroxy-, Methoxi-, Äthoxi-, Propoxi-, Isopropoxi-, N-Butoxi-, Isobutoxi-, sek.-Butoxi- und tert.-Butoxianilin; durch eine Aminogruppe in m- und/oder p-Stellung substituierte Benzoesäurealkylester mit 1 bis 4 Kohlenstoffatomen im Alkylrest, durch eine Aminogruppe in m- und/oder p-Stellung substituierte N-Alkoxicarbonylaminobenzole und -toluole mit 1 bis 4 Kohlenstoffatomen im Alkylrest; $\alpha$- und $\beta$-Naphthylamin; aromatische Diamine, wie 1,3- und 1,4-Diaminobenzol; durch eine Nitro-, Methyl-, Äthyl-, n-Propyl-, Isopropyl-, n-Butyl-, Isobutyl-, sek.-Butyl-, tert.-Butyl-, Methoxy-, Äthoxy-, n-Propoxy-, Isopropoxy-, n-Butoxy-, Isobutoxy-, sel,-Butoxy-, tert.-Butoxygruppe oder ein Halogenatom, vorzugsweise ein Fluor- und/oder Chloratom, in 2- und/oder 4-Stellung substituiertes 1,3-Diaminobenzol oder in 2-Stellung substituiertes 1,4-Diaminobenzol, 1,5- und 1,8-Diaminonaphthalin, 4,4'-Diaminodiphenyl, 2,2'-, 2,4'- und 4,4'-Diamino-diphenylmethan und die entsprechenden Isomerengemische und aromatische Polyamine, wie 1,3,5-Triaminobenzol, 2,4,6-Triaminotoluol, 1,3,5-Triaminonaphthalin, Polyphenyl-polymethylen-polyamine sowie Mischungen aus Diamino-diphenylmethanen und Polyphenyl-polymethylen-polyaminen, die nach bekannten Verfahren durch Kondensation von Anilin und Formaldehyd in Gegenwart von vorzugsweise Mineralsäuren als Katalysatoren erhalten werden.

Besonders bewährt haben sich und daher vorzugsweise verwendet werden als aromatische Monoamine o-, m- und/oder p-Toluidin, o-, m- und/oder p-Anisidin, 3-Hydroxyanilin, o-, m- und/oder p-Chloranilin, 2,4-, 3,4- und 3,5-Dichloranilin, 2-Nitro-4-aminotoluol, 4-Nitro-2-aminotoluol, 2-Nitro-6-amino-toluol und N-alkoxicarbonyl-arylamine der Formel

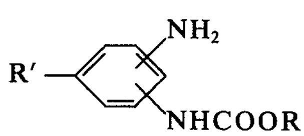

in der R einen Methyl-, Äthyl-, Propyl-, Isopropyl-, n-Butyl-, Isobutyl-, sek.-Butyl- oder tert.-Butylrest und R' ein Wasserstoffatom oder den Rest R bedeuten, sowie insbesondere Anilin, als aromatische Diamine 3,3'-Ditoluylen-4,4'-diamin, 2,4- und 2,6-Toluylendiamin sowie die entsprechenden Isomerengemische, 2,2'-, 2,4'- und 4,4'-Diaminodiphenylmethan und die entsprechenden Isomerengemische, 1,5- und 1,8-Naphthylen-diamin und als Polyamine Mischungen aus Diamino-diphenylmethanen und Polyphenyl-polymethylen-polyaminen.

Bei der Reaktion werden die Aminogruppen in Alkoxicarbonyl-aminogruppen übergeführt, unabhängig davon, ob die übrigen Substituenten unverändert erhalten bleiben oder ebenfalls umgewandelt werden.

Geeignete O-Alkylcarbamidsäureester besitzen die Formel $H_2N-COOR$, in der R einen gegebenenfalls substituierten aliphatischen, cycloaliphatischen oder aromatisch-aliphatischen Rest

bedeutet. In Betracht kommen beispielsweise O-Alkylcarbamidsäureester auf Basis von primären aliphatischen Monoalkoholen mit 1 bis 20 Kohlenstoffatomen, vorzugsweise 1 bis 10 Kohlenstoffatomen, wie Carbamidsäure-methylester, -äthylester, -propylester, -n-butylester, -isobutylester, -2- und -3-methyl-butylester, -neopentylester, -pentylester, -2-methyl-pentylester, -n-hexylester, -2-äthylhexylester, -heptylester, -n-octylester, -n-nonylester, -n-decylester und -n-dodecylester, -2-phenylpropylester und -benzylester, auf Basis von sekundären aliphatischen und cycloaliphatischen Monoalkoholen mit 3 bis 15 Kohlenstoffatomen, vorzugsweise 3 bis 6 Kohlenstoffatomen, wie Carbamidsäure-isopropylester, -sek.-butylester, -sek.-iso-amylester, -cyclopentylester, -cyclohexylester, tert.-butyl-cyclohexylester und -bicyclo-(2,2,1)-heptylester. Vorzugsweise verwendet werden Carbamidsäure-methylester, -äthylester, -propylester, -butylester, -isobutylester, -2- und -3-methylbutylester, -pentylester, -hexylester, -2-äthylhexylester, -heptylester, -octylester und -cyclohexylester.

Als Alkohole können beliebige, gegebenenfalls substituierte primäre oder sekundäre aliphatische Alkohole sowie Mischungen davon verwendet werden. Vorzugsweise wird der den O-Alkylcarbamidsäurestern entsprechende Alkohol eingesetzt.

In Betracht kommen beispielsweise primäre aliphatische Alkohole mit 1 bis 20 Kohlenstoffatomen, wie Methanol, Äthanol, Propanol, n-Butanol, 2-Methyl-butanol, n-Pentanol, Neopentylalkohl, 2-Methyl-pentanol, n-Hexanol, n-Heptanol, n-Octanol, Nonanol, n-Decanol und n-Dodecanol, sekundäre aliphatische und cycloaliphatische Alkohole mit 3 bis 15 Kohlenstoffatomen, vorzugsweise 3 bis 6 Kohlenstoffatomen, wie Isopropanol, sec.-Butanol, sec.-Isoamylalkohol, Cyclopentanol, 2-, 3- oder 4-Methyl-cyclohexanol, Cyclohexanol und Bicyclo-(2,2,1)-heptanol. Vorzugsweise verwendet werden als Monoalkohole Methanol, Äthanol, Propanol, n-Butanol, Isobutanol, 2-Äthyl-butanol, 2- und 3-Methylbutanol, n-Pentanol, n-Hexanol, 2-Äthyl-hexanol, Heptanol, Octanol und Cyclohexanol.

Die Alkohole können gegebenenfalls mit anderen unter den Reaktionsbedingungen inerten organischen Lösungsmitteln gemischt werden.

Zur Herstellung der Aryl-mono-, und/oder -polyurethane nach dem erfindungsgemäßen Verfahren werden die obengenannten primären aromatischen Mono-, und/oder Polyamine mit den O-Alkylcarbamidsäureestern und Alkoholen in solchen Mengen zur Reaktion gebracht, daß das Verhältnis von $NH_2$-Gruppen der primären aromatischen Amine zu O-Alkylcarbamidsäureester zu Alkohol 1 : 0,5 – 20 : 1 – 100, vorzugsweise 1 : 0,8 – 10 : 1 – 50 und insbesondere für Arylmonourethane 1 : 1 – 6 : 1 – 5 und für Aryl-di- und/oder -polyurethane 1 : 1 – 6 : 2 – 30 beträgt.

Wie bereits ausgeführt wurde, wird zur Herstellung der Aryl-mono- und/oder -polyurethane in einer der bevorzugten Verfahrensvarianten neben O-Alkylcarbamidsäureester zusätzlich Harnstoff mitverwendet, wobei das Verhältnis von $NH_2$-Gruppen der aromatischen Amine zur Summe aus O-Alkylcarbamidsäureester und Harnstoff ebenfalls 1 : 0,5 – 20, vorzugsweise 1 : 0,8 – 10 und insbesondere 1 : 1 – 6 beträgt, das Molverhältnis von Harnstoff zu Aminogruppen der primären aromatischen Amine gleich oder kleiner als 1,5, vorzugsweise 1,25 – 0,75 und das Molverhältnis Harnstoff zu Alkohol gleich oder kleiner als 1 sind.

Der Harnstoff wird zweckmäßigerweise in handelsüblicher Form und Reinheit eingesetzt.

Die Umsetzung wird bei erhöhten Temperaturen, beispielsweise bei Temperaturen von 160° bis 300° C, vorzugsweise von 170° bis 230° C und insbesondere von 175° bis 210° C und Drücken von 0,1 bis 120 bar, vorzugsweise 0,5 bis 60 bar, insbesonders 1 bis 40 bar, durchgeführt. Für diesen Temperaturbereich ergeben sich in der Regel Reaktionszeiten von 0,5 bis 100 Stunden, vorzugsweise von 1 bis 50 Stunden und insbesondere von 2 bis 25 Stunden. Bei gegebener Temperatur wird die Reaktion dann vorzugsweise unter einem Druck durchgeführt, bei dem der entstehende Ammoniak selektiv aus dem Reaktionsgemisch abdestilliert werden kann. Die entsprechenden Werte können Tabellen mit physikalischen Kenndaten des Ammoniak und der Alkohole entnommen werden.

Nach dem erfindungsgemäßen Verfahren werden die Aryl-mono- und/oder -polyurethane zweckmäßigerweise wie folgt hergestellt: Die primären aromatischen Mono- und/oder Polyamine, die O-Alkylcarbamidsäureester und Alkohole bzw. vorzugsweise die primären aromatischen Mono- und/oder Polyamine, die O-Alkylcarbamidsäureester, Harnstoff und Alkohole werden in den genannten Mengenverhältnissen gemischt und in einem Reaktionsgefäß, ausgestattet mit einer Vorrichtung zur Abtrennung des Ammoniaks, gegebenenfalls unter Rühren erhitzt. Der entstehende Ammoniak kann nach beendeter Umsetzung abgetrennt werden; vorzugsweise wird er jedoch bereits im Laufe der Umsetzung kontinuierlich oder absatzweise abdestilliert. Hierbei kann es zweckmäßig sein, insbesonders bei der Umsetzung von niedermolekularen Alkoholen unter Druck, den Ammoniak mit Hilfe eines unter den Reaktionsbedingungen inerten Schleppmittels, beispielsweise eines Gases, wie Stickstoff, oder einem Teil des Alkohols abzutrennen.

Aus der erhaltenen Reaktionsmischung wird anschließend, gegebenenfalls nach Abfiltrieren von Feststoffen das Mono- und/oder Polyurethan isoliert, beispielsweise durch Abdestillieren des Alkohols und/oder des Lösungsmittels sowie den gegebenenfalls im Überschuß eingesetzten O-Alkylcarbamaten, durch partielles Abdestillieren des Alkohols und Auskristallisieren, durch Auskristallisieren, durch Ausfällen mit oder auch durch Umkristallisieren aus anderen Lösungsmitteln. Der abgetrennte O-Alkylcarbamidsäureester kann gegebenenfalls zurückgeführt werden.

Die erhaltenen Arylmono- und/oder -polyurethane sind wertvolle End- und Zwischenprodukte. Als Endprodukte werden sie beispielsweise als Schädlingsbekämpfungsmittel verwendet. Als

Zwischenprodukte werden sie beispielsweise als Komponenten für Polykondensations- und Polymersysteme eingesetzt, insbesonders jedoch unter Abspaltung von Alkohol in die entsprechenden Isocyanate übergeführt, wobei Di- und Polyisocyanate zur Herstellung von Polyurethanen Anwendung finden.

Die in den Beispielen genannten Teile beziehen sich auf das Gewicht. Die Elementarzusammensetzungen und Strukturen sind durch Elementaranalyse, Massenspektroskopie sowie JR- und NMR-Spektren bestätigt.

Beispiel 1

20,0 Teile 4,4'-Diamino-diphenylmethan werden mit 44 Teilen Carbamidsäure-n-hexylester in 60 Teilen n-Hexanol 12 Stunden zum Sieden erhitzt, wobei über ein Druckventil im Reaktionsgefäß ein Druck von 2 bis 3 bar eingestellt wird, so daß die Siedetemperatur des Hexanol bei ca. 195° liegt. Der während der Reaktion gebildete Ammoniak wird unter Verwendung von 25 l Stickstoff je 1 Reaktionsgemisch und Stunde als Strippgas kontinuierlich abdestilliert. Beim Erkalten kristallisieren 18,4 Teile 4,4'-Di-(hexoxycarbonylamino)-diphenylmethan (64,0% der Theorie, bezogen auf umgesetztes 4,4'-Diaminodiphenylmethan) mit einem Schmelzpunkt von 142 – 143° C aus $C_{27}H_{38}O_4N_2$ (Molekulargewicht 452). Der Umsatz an 4,4'-Diaminodiphenylmethan beträgt 63,0%. In der Mutterlauge befindet sich noch 4-Amino-4'-(hexoxycarbonylamino)-diphenylmethan.

Beispiel 2

10,0 Teile 4,4'-Diamino-diphenylmethan werden mit 43,7 Teilen Carbamidsäureoctylester in 26,3 Teilen Octanol 2 Stunden lang zum Sieden (200 – 205° C) erhitzt. Danach gibt man weitere 105 Teile Octanol zu der Reaktionsmischung und kocht weitere 15 Stunden bei Rückflußtemperatur, wobei der während der Reaktion gebildete Ammoniak unter Verwendung von 10 l Stickstoff je Liter Reaktionsgemisch und Stunde als Strippmittel kontinuierlich abdestilliert wird. Nach dem Erkalten kristallisieren 7,4 Teile 4,4'-Di-(octoxycarbonylamino)-diphenylmethan (75,6% der Theorie), $C_{31}H_{46}O_4N_2$ (Molekulargewicht 510) vom Schmelzpunkt 117 – 119° C aus. Der Umsatz an 4,4'-Diaminodiphenylmethan ist 38%. In der Mutterlauge befindet sich noch 4-Amino-4'-(octoxycarbonylamino)-diphenylmethan.

Beispiel 3

12,2 Teile 2,4-Diaminotoluol werden mit 22,3 Teilen Carbamidsäureäthylester und 28 Teilen Äthanol 2 Stunden lang zum Sieden erhitzt, wobei über ein Druckventil im Reaktionsgefäß ein Druck von 8 bis 10 bar eingestellt wird, so daß die Siedetemperatur des Äthanols bei 200° C liegt. Der während der Reaktion gebildete Ammoniak wird unter Verwendung von 30 l Stickstoff je Liter Reaktionsgemisch und Stunde als Strippgas kontinuierlich abdestilliert. Danach gibt man weitere 140 Teile Äthanol zu der Reaktionsmischung und kocht weitere 15 Stunden bei Rückflußtemperatur. Nach beendeter Reaktion läßt man abkühlen und stellt das Reaktionsgefäß in eine Eis-Kochsalzmischung, wobei 13,2 Teile (66,6% der Theorie) 2,4-Di-(äthoxycarbonylamino)toluol $C_{13}H_{18}O_4N_2$ (Molekulargewicht 266) vom Schmelzpunkt 105 – 108° C auskristallisieren. Die Hochdruck-Flüssigkchromatographie-Analyse nach der Methode des »Externen Standards« zeigt, daß sich 74,5% des eingesetzten 2,4-Diaminotoluol umgesetzt haben, und daß noch Reste von 2,4-Di-(äthoxycarbonylamino)toluol und ein Gemisch von 2-Amino-4-(äthoxycarbonylamino)toluol und 4-Amino-2-(äthoxycarbonylamino)-toluol in der Mutterlösung vorhanden sind.

Beispiel 4

7,9 Teile 1,5-Diaminonaphthalin werden mit 43 Teilen Carbamidsäureoctylster in 195 Teilen Octanol 20 Stunden zum Sieden (195° C) erhitzt. Nach dem Erkalten kristallisiert ein Niederschlag aus, aus dem durch Umkristallisieren aus Äthylacetat 6,7 Teile (75,0% der Theorie) 1,5-Di-(octoxycarbonylamino)-naphthalin vom Schmelzpunkt 70 – 72° C erhalten werden. Der Umsatz an 1,5-Diaminonaphthalin beträgt 38%.

Beispiel 5

12,2 Teile 2,4-Diaminotoluol werden mit 42,9 Teilen Carbamidsäurecyclohexylester und 200 Teilen Cyclohexanol 15 Stunden zum Sieden erhitzt, wobei über ein Druckventil im Reaktionsgefäß ein Druck

von 2 bis 3 bar eingestellt wird, so daß die Siedetemperatur des Cyclohexanol bei ca. 200° C liegt. Der während der Reaktion gebildete Ammoniak wird unter Verwendung von 15 l Stickstoff je Liter Reaktionsgemisch und Stunde als Strippgas kontinuierlich abdestilliert. Nach dem Erkalten wird das Reaktionsgemisch unter Verwendung der Hochdruck-Flüssigchromatographie nach der Methode des »Externen Standards« analysiert. Hierbei zeigt sich, daß 4,1 Teile (29,6% der Theorie) 2,4-Di-(cyclohexoxycarbonylamino)-toluol und 6,2 Teile (67,6% der Theorie) eines Gemisches aus 2-Amino-4-(cyclohexoxycarbonylamino)toluol und 4-Amino-2-(cyclohexoxycarbonylamino)toluol entstanden sind. Der Umsatz an 2,4-Diaminotoluol beträgt 37%.

Beispiel 6

10,0 Teile eines handelsüblichen Gemisches aus 2,2'-, 2,4'- und 4,4'-Diaminodiphenylmethan und Polyphenyl-polymethylen-polyaminen werden mit 23 Teilen Carbamidsäureäthylester in 30 Teilen Äthanol 15 Stunden zum Sieden erhitzt, wobei über ein Druckventil im Reaktionsgefäß ein Druck von 7 bis 9 bar eingestellt wird, so daß die Siedetemperatur des Äthanols bei ca. 195° C liegt. Der während der Reaktion gebildete Ammoniak wird unter Verwendung von 25 l Stickstoff je Liter Reaktionsgemisch und Stunde als Strippgas kontinuierlich abdestilliert. Man läßt erkalten und destilliert überschüssiges Äthanol und überschüssigen Carbamidsäure-äthylester im Vakuum bis zu einer Sumpftemperatur von 190° C ab. Der Rückstand wird mit 100 Teilen Cyclohexan versetzt und gerührt, wobei man einen pulverförmigen Niederschlag erhält, der abgetrennt und durch Hochdruck-Flüssigchromatographie analysiert wird. Es zeigt sich, daß ein Gemisch aus 2,4'-, 2,2'- und 4,4'-Di-(äthoxycarbonylamino)-diphenylmethan und Polyphenyl-polymethylen-polyäthylurethanen entstanden ist.

Beispiel 7

60 Teile Anilin werden mit 192 Teilen Carbamidsäurebutylester, 39 Teilen Harnstoff und 140 Teilen Butanol 6 Stunden zum Sieden erhitzt, wobei über ein Druckventil im Reaktionsgefäß ein Druck von 5—6,5 bar eingestellt wird, so daß die Siedetemperatur bei ca. 190° C liegt. Der während der Reaktion gebildete Ammoniak wird unter Verwendung von 20 l Stickstoff je l Reaktionsgemisch und Stunde als Schleppmittel kontinuierlich abdestilliert. Nach beendeter Reaktion destilliert man nicht umgesetztes Anilin, überschüssiges Butanol und überschüssigen Carbamidsäurebutylester unter einem verminderten Druck von ca. 20 mbar ab und erhält anschließend durch Destillation bei 0,05 mbar und 140—42° C 87 Teile Phenylbutylurethan (94,4% der Theorie, bezogen auf umgesetztes Anilin). Der Umsatz an Anilin beträgt 74%.

Beispiel 8

93 Teile Anilin werden mit 350 Teilen Carbamidsäuremethylester und 96 Teilen Methanol 6 Stunden lang auf 175° C erhitzt, wobei über ein Druckventil im Reaktionsgefäß ein Druck von 5—6 bar eingestellt wird. Der während der Reaktion gebildete Ammoniak wird portionsweise abdestilliert. Nach beendeter Reaktion unterwirft man die Reaktionsmischung der fraktionierten Destillation, wobei neben nicht umgesetztem Anilin, überschüssigem Methanol und Carbamidsäuremethylester 109 Teile Phenylmethylurethan (85% der Theorie, bezogen auf umgesetztes Anilin) vom Schmp. 54—55° erhalten werden. Der Umsatz des Anilin beträgt 85%.

Beispiel 9

120 Teile 3,5-Dichloranilin werden mit 280 Teilen Carbamidsäuremethylester und 75 Teilen Methanol 18 Stunden lang auf 185° C erhitzt, wobei über ein Druckventil im Reaktionsgefäß ein Druck von 9—10 bar eingestellt wird. Der während der Reaktion gebildete Ammoniak wird unter Verwendung von 30 l Stickstoff je l Reaktionsgemisch und Stunde als Schleppmittel kontinuierlich abdestilliert. Nach beendeter Reaktion wird die Reaktionslösung gaschromatographisch nach der Methode des »Internen Standard« analysiert. Es zeigt sich, daß 94 Teile 3,5-Dichlorphenylmethylurethan (83,6% der Theorie, bezogen auf umgesetztes 3,5-Dichloranilin) vom Schmp. 118—20° gebildet werden. Der Umsatz des 3,5-Dichloranilin beträgt 69%.

Beispiel 10

15,2 Teile 2-Amino-4-nitrotoluol werden mit 40 Teilen Carbamidsäuremethylester und 10 Teilen

Methanol 20 Stunden lang auf 190° C erhitzt, wobei über ein Druckventil im Reaktinsgefäß ein Druck von 7 – 9 bar eingestellt wird. Der Während der Reaktion gebildete Ammoniak wird unter Verwendung von 15 l Stickstoff je l Reaktionsgemisch und Stunde als Schleppmittel kontinuierlich abdestilliert. Nach beendeter Reaktion wird die Reaktionslösung durch Hochdruck-Flüssigchromatographie nach der Methode des »Externen Standard« analysiert. Es zeigt sich, daß 15 ATeile 2-Methoxicarbonylamino-4-nitrotoluol (96,5% der Theorie, bezogen auf umgesetztes 2-Amino-4-nitrotoluol) vom Schmp. 133 – 34° gebildet werden. Der Umsatz des 2-Amino-4-nitrotoluol beträgt 74%.

Beispiel 11

21 Teile 4-Aminotoluol werden mit 11,8 Teilen Harnstoff, 70 Teilen Carbamidsäureäthylester und 36 Teilen Äthanol 10 Stunden auf 185° C erhitzt, wobei über ein Druckventil im Reaktionsgefäß ein Druck von 7 – 9 bar eingestellt wird. Der während der Reaktion gebildete Ammoniak wird unter Verwendung von 20 l Stickstoff je l Reaktionsgemisch und Stunde als Schleppmittel kontinuierlich abdestilliert. Nach beendeter Rekation destilliert man nicht umgesetztes 4-Aminotoluol, überschüssiges Äthanol und überschüssigen Carbamidsäureäthylester unter einem verminderten Druck von ca. 18 mbar ab und erhält durch weitere Destillation bei 0,1 mbar und 125 – 30° C 30 Teile 4-Äthoxicarbonylaminotoluol (97% der Theorie, bezogen auf umgesetztes 4-Aminotoluol) vom Schmp. 55 – 57°. Der Umsatz an 4-Aminotoluol beträgt 88%.

Beispiel 12

22 Teile 3-Aminophenol werden mit 100 Teilen Carbamidsäureäthylester, 12 Teilen Harnstoff und 45 Teilen Äthanol 10 Stunden lang auf 185° C erhitzt, wobei über ein Druckventil im Reaktionsgefäß ein Druck von 7 – 8 bar eingestellt wird. Der während der Reaktion gebildete Ammoniak wird kontinuierlich unter Verwendung von 20 l Stickstoff je l Reaktionsgemisch und Stunde als Schleppmittel abdestilliert. Nach beendeter Reaktion destilliert man überschüssiges Äthanol und überschüssigen Carbamidsäureäthylester bei einem Unterdruck von ca. 15 mbar ab, gießt den Rückstand in soviel 10prozentige Natronlauge bis alles gelöst ist und stellt die Lösung anschließend mit 10prozentiger Schwefelsäure auf pH 5 ein. Es fallen 23,3 Teile 3-Äthoxycarbonylaminophenol (63,8% der Theorie, bezogen auf eingesetztes 3-Aminophenol) vom Schmp. 99 – 100° aus.

Beispiel 13

10 Teile 4,4'-Diaminodiphenylmethan werden mit 15,2 Teilen Carbamidsäuremethylester und 6 Teilen Harnstoff in 35 Teilen Methanol 6 Stunden lang auf 185 – 190° C erhitzt, wobei über ein Druckventil im Reaktionsgefäß ein Druck von 24 – 27 bar eingestellt wird. Der während der Reaktion gebildete Ammoniak wird unter Verwendung von 30 l Stickstoff je l Reaktionsgemisch und Stunde als Schleppmittel kontinuierlich abdestilliert. Nach dem Erkalten kristallisieren 14 Teile 4,4'-Di-(methoxicarbonylamino)-diphenylmethan (88,3% der Theorie, bezogen auf umgesetztes 4,4'-Diaminodiphenylmethan) vom Schmp. 184 – 185° aus. Der Umsatz an 4,4'-Diaminodiphenylmethan ist praktisch quantiativ. In der Mutterlauge befindet sich noch 4-Amino-4'-(methoxicarbonylamino)-diphenylmethan.

Beispiel 14

10 Teile 4,4'-Diaminodiphenylmethan werden mit 15,2 Teilen Carbamidsäuremethylester in 65 Teilen Methanol 18 Stunden zum Sieden erhitzt, wobei über ein Druckventil im Reaktionsgefäß ein Druck von 35 bis 37 bar eingestellt wird, so daß die Siedetemperatur des Methanols bei 180 – 190° C liegt. Der während der Reaktion gebildete Ammoniak wird unter Verwendung von 30 l Stickstoff je Liter Reaktionsgemisch und Stunde als Strippgas kontinuierlich abdestilliert. Nach dem Erkalten kristallisieren 10,6 Teile (83,6% der Theorie) 4,4'-Di-(methoxycarbonylamino)-diphenylmethan $C_{17}H_{18}O_4N_2$ (Molekulargewicht 314) vom Schmelzpunkt 184 – 186° C aus. Es haben sich 80% 4,4-Diaminodiphenylmethan umgesetzt.

**Patentansprüche**

1. Verfahren zur Herstellung von Aryl-mono- und/oder -polyurethanen, dadurch gekennzeichnet, daß man O-Alkylcarbamidsäureester mit primären aromatischen Mono- und/oder Polyaminen in Gegenwart von Alkoholen und gegebenenfalls Harnstoff bei erhöhten Temperaturen umsetzt und den

entstehenden Ammoniak gegebenenfalls abtrennt.

2. Verfahren nach Anspruch 1, dadurch gekennzeichnet, daß man die Ausgangskomponenten in solchen Mengen unmsetzt, daß das Molverhältnis von $NH_2$-Gruppen der aromatischen Mono- und/oder Polyamine zu O-Alkylcarbamidsäureester zu Alkohol 1 : 0,5 – 20 : 1 bis 100 beträgt.

3. Verfahren nach Anspruch 1, dadurch gekennzeichnet, daß man neben O-Alkylcarbamidsäureestern zusätzlich Harnstoff mitverwendet, wobei das Molverhältnis von Harnstoff zu Alkohol gleich oder kleiner als 1 ist.

4. Verfahren nach Anspruch 3, dadurch gekennzeichnet daß man neben O-Alkylcarbamidsäureester Harnstoff einsetzt, wobei das Molverhältnis von Harnstoff zu Aminogruppen der primären aromatischen Mono- und/oder Polyamine gleich oder kleiner als 1,5 ist.

5. Verfahren nach Anspruch 1, dadurch gekennzeichnet, daß man als primäre aromatische Monoamine Anilin 3-Hydroxyanilin, 3,5-Dichloranilin und als Polyamine 2,4- und 2,6-Diamino-toluol und die entsprechenden Isomerengemische, 1,5-Diaminonaphthalin, 3,3'-Ditoluylen-4,4'-diamin, 2,2'-, 2,4'- und 4,4'-Diamino-diphenylmethan und die entsprechenden Isomerengemische und Mischungen aus Diamino-diphenylmethanen und Polyphenylpolymethylen-polyaminen verwendet.

6. Verfahren nach Anspruch 1, dadurch gekennzeichnet, daß man als O-Alkylcarbamidsäureester solche aus Carbamidsäure und aliphatischen Monoalkoholen mit 1 bis 10 Kohlenstoffatomen und cycloaliphatischen Monoalkoholen mit 3 bis 6 Kohlenstoffatomen im Alkkoholrest verwendet.

7. Verfahren nach Anspruch 1, dadurch gekennzeichnet, daß man als Alkohole die den O-Alkylcarbamidsäureestern entsprechenden Alkohole verwendet.

8. Verfahren nach Anspruch 1, dadurch gekennzeichnet, daß man die Umsetzung bei Temperaturen von 160° – 300° C durchführt.

9. Verfahren nach Anspruch 1, dadurch gekennzeichnet, daß man die Umsetzung bei Drücken von 0,1 bis 120 bar durchführt.

10. Verfahren nach Anspruch 1, dadurch gekennzeichnet, daß man gleichzeitig den entstehenden Ammoniak abtrennt.

## Claims

1. A process for the preparation of an aryl mono- and/or polyurethane, wherein O-alkyl carbamates are reacted with primary aromatic mono- and/or polyamines, at elevated temperature, in the presence of alcohols and in the presence or absence of urea, and the ammonia which is formed is, if desired, separated off.

2. A process as claimed in claim 1, wherein the starting components are reacted in such quantities that the molar ratio of $NH_2$ groups of the aromatic mono- and/or polyamines to O-alkyl carbamate to alcohol is 1 : 0.5 – 20 : 1 – 100.

3. A process as claimed in claim 1, wherein urea is employed in addition to O-alkyl carbamates, the molar ratio of urea to alcohol being equal to or less than 1.

4. A process as claimed in claim 3, wherein urea is employed in addition to O-alkyl carbamates, the molar ratio of urea to amino groups of the aromatic mono- and/or polyamines being equal to or less than 1.5.

5. A process as claimed in claim 1, wherein aniline, 3-hydroxyaniline or 3,5-dichloraniline are used as primary aromatic monoamines, and 2,4- and 2,6-diaminotoluene, the corresponding isomer mixtures thereof, 1,5-diaminonaphthalene, 3,3'-ditoluene-4,4'-diamine, 2,2'-, 2,4'- and 4,4'-diaminodiphenylmethane and the corresponding isomer mixtures thereof, and mixtures of diaminodiphenylmethanes and polyphenylpolymethylene-polyamines are used as polyamines.

6. A process as claimed in claim 1, wherein the O-alkyl carbamates used are those of carbamic acid and aliphatic monoalcohols having 1 to 10 carbon atoms in the alcohol radical and cycloaliphatic monoalcohols having 3 to 6 carbon atoms in the alcohol radical.

7. A process as claimed in claim 1, wherein the alcohols used are the same as those employed in the preparation of the O-alkyl carbamates.

8. A process as claimed in claim 1, wherein the reaction is carried out at temperatures of from 160° C to 300° C.

9. A process as claimed in claim 1, wherein the reaction is carried out at pressures of from 0.1 bar to 120 bars.

10. A process as claimed in claim 1, wherein the ammonia is simultaneously separated off as it is formed.

## Revendications

1. Procédé de préparation d'aryl-mono- et/ou polyuréthanes, caractérisé par le fait qu'on fait réagir, à température élevée, un ester d'acide O-alkylcarbamique avec des mono- et/ou polyamines aromatiques primaires, en présence d'alcools et éventuellement d'urée et on sépare éventuellement

l'ammoniac formé.

2. Procédé selon la reyendication 1, caractérisé par le fait que l'on fait réagir les composants de départ en quantités telles que les rapports molaires entre les groupes $NH_2$ des mono- et/ou polyamines aromatiques, l'ester d'acide O-alkylcarbamique et l'alcool soient de 1/0,5 à 20/l à 100.

3. Procédé selon la revendication 1, caractérisé par le fait qu'à côté de l'ester d'acide O-alkylcarbamique on utilise en plus de l'urée, le rapport molaire entre l'urée et l'alcool étant égal ou plus petit que 1.

4. Procédé selon la revendication 3, caractérisé par le fait qu'à côté d'ester d'acide O-alkylcarbamique on fait réagir de l'urée, le rapport molaire entre l'urée et les groupes amino de la mono- et/ou polyamine aromatique primaire étant égal ou inférieur à 1,5.

5. Procédé selon la revendication 1, caractérisé par le fait que l'on utilise, comme monoamine aromatique primaire, aniline 3-hydroxyaniline, 3,5-dichloraniline et comme polyamine, 2,4- et 2,6-diamono-toluène et les mélanges isomères correspondants, 1,5-diaminonaphtalène, 3,3'-ditoluylen-4,4'-diamine, 2,2'-, 2,4'- et 4,4'-diaminodiphénylméthane et les mélanges isomères correspondants et des mélanges de diamino-diphénylméthane et polyphénylpolyméthylène-polyamine.

6. Procédé selon la revendication 1, caractérisé par le fait que l'on utilise, comme esters d'acide O-alkylcarbamique, ceux d'acide carbamique et de monoalcools aliphatiques à 1 à 10atomes de carbone et de monoalcools cycloaliphatiques à 3 à 6 atomes de carbone dans le reste alcool.

7. Procédé selon la revendication 1, caractérisé par le fait l'on utilise, comme alcools, les alcools correspondant aux esters d'acide O-alkylcarbamique.

8. Procédé selon la revendication 1, caractérisé par le fait que l'on effectue la réaction à des températures de 160° à 300° C.

9. Procédé selon la revendication 1, caractérisé par le fait que l'on effectue la réaction à des pressions de 0,1 à 120 bars.

10. Procédé selon la revendication 1, caractérisé par le fait que l'on sépare simultanément l'ammoniac formé.